# EUROPEAN PATENT SPECIFICATION

(11) **EP 1 009 418 B1**
(45) Date of publication and mention of the grant of the patent: **27.03.2002**
(21) Application number: 98940437.1
(22) Date of filing: 28.08.1998
(51) Int. Cl.: A61K 35/78, A61K 38/16

(54) **ROBINIA PSEUDOACACIA LECTIN AND ITS USES**
ROBINIA PSEUDOACACIA LEKTIN UND SEINE VERWENDUNGEN
LECTINE DE ROBINIA PSEUDOACACIA ET SES UTILISATIONS

(30) Priority: 29.08.1997 GB 9718413
(43) Date of publication of application: 21.06.2000
(73) Proprietor: Alizyme Therapeutics Limited, Milton Road, Cambridge CB4 4WE (GB)
(72) Inventor: PUSZTAI, Arpad Janos, Rowett Research Institute, Bucksburn, Aberdeen AB2 9SB (GB); BARDOCZ, Zsuszanna Magdolna, Rowett Res. Services, Bucksburn, Aberdeen AB2 9SB (GB); PALMER, Richard Michael John, Alizyme Thera. Ltd., Cambridge CB4 4WE (GB); FISH, Neil William, Alizyme Therapeutics Limited, Cambridge C4 4WE (GB)
(74) Representative: Cornish, Kristina Victoria Joy
(86) International application number: GB9802612
(87) International publication number: WO9911278

(56) References cited:
- DATABASE WPI Section Ch, Week 9419 Derwent Publications Ltd., London, GB; Class B04, AN 94-157940 XP002086657 & SU 1 799 599 A (LVOV MED INST) , 7 March 1993
- DATABASE WPI Section Ch, Week 8704 Derwent Publications Ltd., London, GB; Class B04, AN 87-024774 XP002086658 & JP 61 280432 A (ASAHI CHEM IND CO LTD) , 11 December 1986
- JOSIANE WANTYGHEM ET AL.: "PURIFICATION AND CHARACTERIZATION OF ROBINIA PSEUDOACACIA SEED LECTINS" THE BIOCHEMICAL JOURNAL, vol. 237, no. 2, 15 July 1986, pages 483-489, XP002086656 cited in the application

## Description

The present invention relates to *Robinia pseudoacacia* (RPA) lectin as defined in the claims for use in medicine and to the use of *Robinia pseudoacacia* lectin in the manufacture of a medicament for the reduction and/or treatment of disease as defined in the claims.

Lectins are proteins or glycoproteins which are characterised by their ability to recognise specific glycoconjugate structures (Pusztai 1991). Some orally ingested lectins are resistant to digestion in the stomach and survive intact to the intestine. Within the gut, lectins may attach to carbohydrate structures expressed on the surface of various gut mucosal cells (Pusztai *et al* 1995). The consequence of lectin attachment to gut cells may vary. For example, biological responses may depend on the administered dose. At high doses, kidney bean lectin (PHA) causes a reversible disorganisation of epithelial cell villi. This is quickly repaired by endogenous processes. Similarly, in the presence of large amounts of PHA in the small intestine an overgrowth of endogenous *Escherichia coli* occurs. In addition, lipid mobilisation and glucose oxidation are increased and protein synthesis in skeletal muscle is reduced. However, at lower doses of PHA, an enhanced rate of crypt cell production, resulting in hyperplastic growth in the gut, is observed (Bardocz *et al* 1995), but without the damaging effects which occur at high doses.

*Robinia pseudoacacia* (black locust) is a leguminous tree species that is common in North America and Central Europe. *Robinia pseudoacacia* produces lectins in its bark, roots, root nodules, phloem, wood and leaves (Geitl and Ziegler 1980) and in the seeds. (Van Damme *et al* 1995b). Although the precise function of the lectins is unknown, it is believed that the lectins may be used as a store for nitrogen in dormant periods (Yoshida *et al* 1994). It is also known that the level of lectin within the bark increases during winter. Given the known toxicity of Robinia lectins to mammals, (Nishiguchi *et al* 1997) it is also possible that the bark lectin may act as a defence mechanism against herbivores such as rabbits during winter.

The bark and seed lectins of *Robinia pseudoacacia* have been characterised at both the biochemical and molecular levels (Van Damme *et al*., 1995a, 1995b). Root lectin has also been isolated and characterised (Duverger *et al* 1997). *Robinia pseudoacacia* lectins (predominantly seed derived) have had a number of uses, primarily as diagnostic and research tools. The specific carbohydrate binding properties (N-acetyl-D-galactosamine) of RPA have been used to investigate the carbohydrate composition of biological tissue (Raedler *et al* 1982). The mitogenic properties of RPA have also been used in *in vitro* studies to investigate the biology of isolated lymphocytes (Sabeur *et al* 1986, Sharif *et al* 1978 & 1977). Banach *et al* (1983) showed that intraperitoneal doses of RPA in mice induced a hepatotoxic effect including a rapid fall in liver glycogen. Pusztai (1993) also showed that RPA bound strongly to the gut wall of the rat and induced pancreatic growth at high doses suggesting that RPA may damage the gut wall, promote coliform overgrowth and induce pancreatitis.

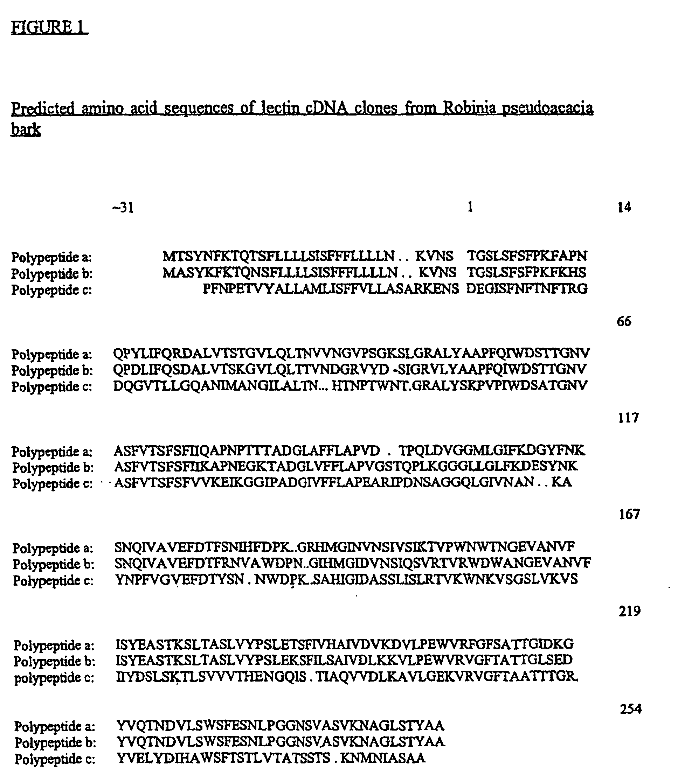

The lectins from the bark of *Robinia pseudoacacia* can be characterised using a range of gel separation. DNA and protein sequencing techniques (Van Damme et al 1995a). Native bark lectin eluted from a column consists of two lectins, RPbAI and RPbAII which co-purify at an apparent molecular weight of 120 kDa. Analysis of the RPbAI on an SDS PAGE gel indicates that it is composed of two subunits, polypeptide a and polypeptide b, with molecular weights of 31.5 and 29 kDa respectively. RPbAI is a tetramer composed of all the possible combinations of the a and b polypeptide monomers. Polypeptides a and b have been further characterised by cDNA cloning and sequencing using standard techniques. The predicted protein sequences of polypeptides a and b are shown in Figure 1.

RPbAII is a tetramer composed of a single monomer polypeptide (subunit) c of 26 kDa molecular weight. Polypeptide c has been further characterised and the cDNA sequenced and the predicted protein sequence is also shown in Figure 1.

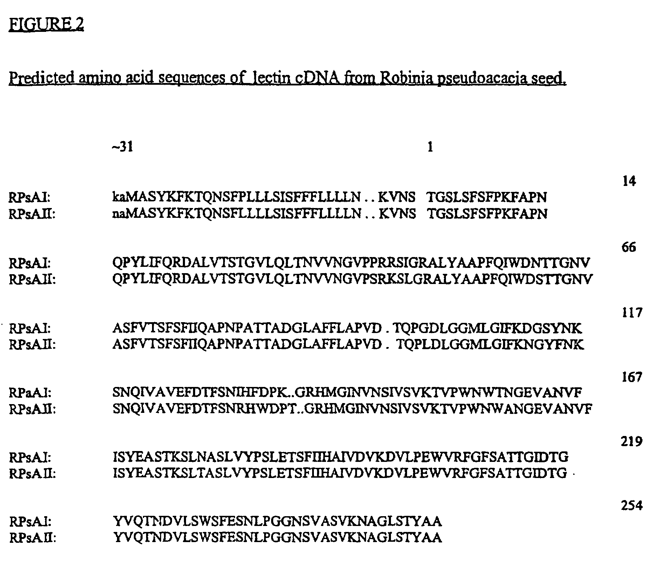

*Robinia pseudoacacia* seed contains two lectins, RPsAI and RPsAII which co-purify at an apparent molecular weight of 120 kDa (Van Damme *et al*, 1995b). Analysis of both lectins on SDS PAGE indicates that they are each comprised of a single monomer subunit (34 and 29 kDa respectively). The seed lectins have been characterised by cDNA cloning and sequencing techniques and the predicted protein sequence is shown in Figure 2. Recently published work on the root lectins has shown that they are composed of two lectins (Duverger *et al*, 1997). The root lectins appear to differ from those of seed and bark in that they are dimer molecules classified as RPrAI and RPrAII of molecular weights 58 and 63 kDa respectively. RPrAI is a heterodimer consisting of a 31 and a 29 kDa subunit and RPrAII is a homodimer consisting of single 30 kDa subunits.

Studies on *Robinia pseudoacacia* lectins have generated some confusion concerning the precise molecular weight, and subunit composition of the individual lectins, Wantyghem *et al* (1986) identified two lectins in seeds, RPA 1 a dimer and RPA 3 a tetramer. However, in the same year Fleischmann *et al* (1986) described the isolation of two tetramer lectins termed RPA 1 and RPA 2 with similar subunit molecular weights. It appears therefore that the lectins may exist as monomer, dimer, trimer and tetramer forms.

Mucosal cells make up mucous membranes which are the moist membrane lining many tubular structures and cavities of living bodies. They include the mucous covering of the gut, the mouth, the nasal passage, the oesophagus, the stomach. the lung, the small intestine, and the large intestine (including the rectum). The importance of mucous membranes in the viability of an organism has long been recognised. However, the ability to control mucosal and non-mucosal cell proliferation has never previously been recognised as an opportunity to address many physiological problems, including diseased and damaged cells and/or tissues. A number of disorders of mucosal cells are known, including conditions where cell division is accelerated, decelerated. where the mucosal cells are damaged or where the protective outer layer such as mucous is missing. Conditions related to the abnormal control of mucosal cell proliferation may include skin cancers, psoriasis irritable bowel syndrome, inflammatory disease and mucositis.

Mucositis is a painful and debilitating condition in which the rapidly growing epithelial and mucosal cells are damaged and the external mucous layer is removed and/or not replaced sufficiently quickly. Mucositis may result in infection by microorganisms which are present, for example, in the mouth or gut. In this text, mucositis covers all gut lesions (which may occur as an after-effect of damage to the mucous membrane). The condition is seen as a major side effect in the treatment of cancer. The incidence and severity of mucositis may increase with increasing rounds of cancer therapy, and may ultimately affect treatment compliance and survival. The solution which the present invention provides exploits the tissue protecting qualities and the metabolic effects of lectins on biological material.

Diseased and damaged cells, which cannot repair or regenerate sufficiently quickly can cause serious and potentially life-threatening health risks. Also a problem in maintaining normal cell functions are metabolic diseases. such as obesity. hyperglycemia, cardiovascular disease, stroke and gastrointestinal disease, including irritable bowel syndrome, inflammatory bowel disease and coeliac disease.

Cells and tissues can become diseased and damaged through a wide variety of causes. Cell damaging agents include: chemotherapeutic agent(s), radiotherapy, chemical(s) (organic or inorganic), toxin(s), acid, alkali, a radiation source, a free radical, and may cause damage, by surgery or therapy in an attempt to treat an alternative, more problematic disease. The treatment of cancer is a prime example of damage caused to cells and/or tissues of an organism as a side-effect of other procedures. The treatment of cancer is based primarily upon the use of chemotherapy or radiotherapy, either alone, together or in combination with surgery. Chemotherapy uses a cytotoxic agent to directly damage the DNA of a target cell. If a sufficient dose of the cytotoxic agent is administered to a target tissue i.e. a tumour, DNA mis-repair may result in the accumulation of DNA mutations, lesions and chromosomal aberrations that ultimately result in cell death. Radiotherapy uses radiation to either damage the DNA of a target cell directly, or exploits the potential of ionising radiation to produce free radicals which are able to break DNA strands (Steel, 1996). The use of chemotherapy or radiotherapy is therefore a compromise in trying to induce most damage to the cancerous cells by targeting the radiation without irreparably destroying normal tissue.

Although chemotherapy and radiotherapy are the most widely used treatments for cancer, the rates of survival are limited due to a number of factors. The key factor is that the cytotoxic drug or radiation does not discriminate between normal and cancerous tissue. In most cases, it is impossible to give a sufficient dose of cvtotoxic drug or radiation to reliably kill all cancerous tissue as it would prove fatal to the patient. Common side effects for existing therapy regimes include hair loss, bone marrow suppression, nausea, vomiting and diarrhoea (Paulsen *et al* 1996). In addition, there are also many instances where the use of radiotherapy, particularly to the pelvic regions has resulted in altered gastrointestinal function (Yeoh *et al* 1993) and long term damage to the gut which requires surgery (van Halteren *et al* 1993).

A major breakthrough has been made in the last 10 years with the availability of hematopoietic growth factors. It is now possible to give higher doses of cytotoxic drugs and then rescue tissues such as bone marrow and white blood cells by the administration of recombinant growth factors such as granulocyte-macrophage colony-stimulating factor (Erkisis et al, 1996). Such an approach has enabled improved prognosis and survival rates to be achieved. Whilst epidermal-specific growth factors such as epidermal growth factor are known, the complex nature of gut growth regulation has made it difficult to develop effective gut "rescue" procedures (Podolsky, 1993). As no such growth factor currently exists for the gut, damage to the gastointestinal tract by cytotoxic drugs and radiation has now become dose limiting.

The present invention overcomes problems in the art by providing a new and effective product and the use thereof which enables positive and controlled growth in and of biological material.

Accordingly, one aspect of the invention provides *Robinia pseudoacacia* lectin as defined in the claims for use in medicine. The use in medicine is widespread. It includes the control of mucosal cell proliferation, the reduction and/or treatment of damage caused by a cell-darnaging agent, the reduction and/or treatment of a metabolic disorder, or a combination of two or more thereof. Throughout this text, "reduction" means any effect which mitigates any damage or any medical disorder, to any extent, and includes prevention (prophylactic use). Throughout this text, "treatment" means any amelioration of disorder, disease, syndrome, condition, pain or a combination of two or more thereof. Treatment by the present invention (or use of compositions) may be carried out during an additional "treatment", such as chemotherapy and/or radiotherapy.

*Robinia pseudoacacia* lectin is particularly useful in the control of mucosal cell proliferation as it relates to the reduction and/or treatment of mucositis, including gut lesions. Control of mucosal cell proliferation is particularly useful in the reduction and/or treatment of damage caused by a cell-damaging agent. Typical cell damaging agents of all aspects of the present invention include radiotherapy, chemotherapy or a combination thereof. In the present application the terms ''radiation" and "radiotherapy" are used as having the same meaning, which is a source of radiation (irradiation) which may, or may not be applied as a therapeutic technique.

The invention is of particular applicability to the reduction and/or treatment of damage to mammalian tissue, more particularly human tissue. The present invention is of particular importance to human tissue because of the considerable requirement for radiotherapy and/or chemotherapy in the treatment of cancer. However, the present invention is also applicable to other animals and biological matter, in particular to the veterinary industry, including farm animals and pets.

*Robinia pseudoacacia* for use in medicine relates to all cells and/or tissues, and includes cells and tissues within a whole body as well as outside of a whole body, including isolated cells and tissues. It relates to all biological matter, including whole bodies and parts thereof. The invention also relates to matter which is subjected, intentionally or unintentionally, to any cell-damaging agent.

The invention is of particular use to biological matter which is particularly sensitive to cell-damaging agents such as radiotherapy and/or chemotherapeutic agents. Such biological matter includes mucous membranes, in particular the mucosal coverings of the gut, the mouth, the nasal passage, the oesophagus, the stomach. the lung, the small intestine, the large intestine (including the colon and the rectum), epithelial tissue (eg covering the eye) as well as any other mucosal cell and/or tissue and non-mucosal cells and tissues such as bone marrow, spleen, all blood generating cells, blood tissue, thymus, hair-producing tissue, eye tissue, reproductive tract, testicular/prostate tissue or two or more thereof. The present invention is particularly relevant to mucous membranes which are tissues lining hollow structures in the body and include the permanently moist tissues lining the eyes. The sensitivity of cells and tissues to damage by a cell-damaging agents may be linked to its metabolic status. It is the growth factor effect of *Robinia pseudoacacia* lectin, in particular on the gut (and especially the small intestine) which is understood to be important in the prophylactic and/or therapeutic affects prior to, during and/or following administration of cell-damaging agents.

*Robinia pseudoacacia* lectin is particularly effective in the control of mucosal cell proliferation prior to, during and/or following radiotherapy, chemotherapy or a combination thereof. The invention is achieved as a result of the protective and repair capabilities of *Robinia pseudoacacia* lectin.

Mucosal cells are those which make up any mucous membrane (the moist membrane lining many tubular structures and cavities). Many are those which provide a protective layer between the external environment and the internal organs of an animal. Mucosal cells/tissues include mucosal coverings of the gut, the mouth, the nasal passage, the oesophagus, the stomach; the lung, the small intestine, the large intestine, epithelial tissue, reproductive tract and others. The first aspect of the invention relates to all of these. Other cells and tissues to which the first aspect of the invention relates include; bone marrow, spleen, blood generating cells, blood tissue, thymus, hair-producing tissue. eye tissue, the reproductive tract. testicular/prostate tissue and combinations of two or more thereof.

In accordance with the present invention, the use of *Robinia psuedoacacia* lectin in medicine may be in combination with a cytoprotectant. The cytoprotectant is preferably a radiosensitiser, a chemoprotectant (including free-range scavangers), a growth factor or a combination of two or more thereof. From the above list of cytoprotectants the following examples are preferred: radiosensitisers- vitamin K mimetics such as Synkavit or Menadione, gadolinium texaphyrin or iobenguane (([[3-iodo-13311)phenyl]methyl]guanidine): chemoprotectants- Sulcraphate, cysteine, cysteamine, Ethyol, balazipone or dosmalfate; free radical scavengers - WR 3689 (2-[[3-methylamino)propyl]amino]ethanediol dihydrogen phosphate ester, AD 20 (2-[[2-methoxyphenyl)acetyl]amino]-2-propenoic acid or nitroxide antioxidant; growth factors-granulocyte colony-stimulating factor (G-CSF), granulocyte-macrophage colony-stimulating factor (GM-CSF), Erythropoietin (EPO), epidermal growth factor (EGF), keratinocyte growth factor (KGF), transforming growth factor (TGF α and β), any interleukin including IL-11 and IL-15, insulin-like growth factor (IGF), nerve growth factor (NGF), platelet derived growth factor (PDGR), Bombesin, Relaxin, Calcitonin. colostrum-derived growth factor (CDGF), amlexanox or amoxanox, protegrin, pilocarpine hydrochloride, stem cell factor (STF), thrombopoietin, steel factor (SF), any interferon, including interferon or any cytokine.

In addition to cytoprotectants, the use of the lectin may be in combination with one or more other compounds/agents (preferably pharmaceutical). In particular, anti-microbial agents may be included. Such agents may be included to fight secondary infections, for example secondary infections associated with mucositis.

The *Robinia pseudoacacia* lectin for use in medicine is particularly useful in relation to cells of the gastrointestinal tract (for both reduction and repair of damage and in relation to metabolic disease). The control may be for an increase in functional and/or length of the gastrointestinal tract or for control of the nature and/or density of gastrointestinal-cell expressed surface glycoproteins. Other uses in relation to control of mucosal cell proliferation include the reduction and/or treatment of bowel disorders such as inflammatory bowel disease and irritable bowel syndrome as well as the reduction and/or treatment of gut lesions, and the repair and replacement of mucosal cells prior to, during or following, radiotherapy, chemotherapy or a combination of two or more thereof.

The applications of these control features includes:
(a) Gut cell proliferation leading to an increase in functional gut area and length which could be a useful therapy where gut function is impaired as for instance via surgery or accident, and/or
(b) Control of gut cell turnover rates which may allow control over the nature and density of expressed surface glycoconjugates as these become progressively more complex as cells age. As the glycoconjugates affect certain gut properties. such as propensity to bacterial attachment, lectin administration could be envisaged as a therapeutic or prophylactic control for these properties.

In particular, the control of cell proliferation may be used for obtaining an increased nutrient capacity of the small intestine and/or controlling cell gut expression of glycoconjugates. Such effects are not necessarily medical disorders and may be only cosmetic or functional, above and beyond the satisfactory medical level.

In accordance with the invention, the higher the lectin concentration, the faster may be the reduction and/or treatment of any medical disease or other non-medical symptoms, as defined in the claims. However, various factors may influence the preferred concentration of lectin dosage, as discussed herein.

In the present invention the *Robinia pseudoacacia* lectin may be those in either naturally occurring compositions or purified. therefrom, chemically synthesised or produced by recombinant technology. The lectin may be modified or a derivative (naturally occurring or synthetic). Methods are well known in the art for the production of lectins, both purification from natural sources and by recombinant technology (Pusztai and Palmer, 1977, Carvalho, 1993 and US Patent no. 4,889,842). The lectins according to the invention are preferably functional equivalents to "originally" isolated *Robinia pseudoacacia* lectins. The functionality of any *Robinia pseudoacacia* lectin can be determined in accordance with its ability to attach to specific glycoconjugate structures. Most preferably, a polypeptide is a *Robinia pseudoacacia* lectin according to the present invention if it is an equivalent (or lectin variant as defined herein after), i.e., 40%, preferably 50%, preferably 6.0%, more preferably 70%, even more preferably, 80% or more identical at the amino acid level or DNA sequence level to any known *Robinia pseudoacacia* lectin, for example the lectins set out in Figure 1 or 2.

A "lectin variant" as referred to herein, means a polypeptide substantially homologous to native lectin, but which has an amino acid sequence different from that of native lectin (human, murine or other mammalian species) because of one or more deletions, insertions or substitutions. The variant amino acid sequence preferably is at least 80% identical to a native lectin amino acid sequence, most preferably at least 90% identical. The percent identity may be determined, for example, by comparing sequence information using the GAP computer program, version 6.0 described by Devereux *et al.* (*Nucl. Acids Res.* 12:387, 1984) and available from the University of Wisconsin Genetics Computer Group (UWGCG). The GAP program utilises the alignment method of Needleman and Wunsch (*J. Mol. Biol.* 48:443, 1970), as revised by Smith and Waterman (*Adv. Appl. Math* 2:482, 1981).

Since lectins are proteins, they are clearly subject to destabilisation/denaturation by a number of parameters such as heat, acid, alkali etc. Since the use of lectins in the present invention (throughout) requires their protein properties it is important that the lectins are not completely destroyed or denatured prior to or during their use (eg in the strong acid conditions of the stomach and the mild alkali conditions of the lower gut). Accordingly, the *Robinia pseudoacacia* lectins for use in the present invention may need to be first characterised with regard to how they may be affected during processing and/or administration. Such characterisation is standard technology and can be conducted easily by any person skilled in the art. The concentration of lectin required for any of the aspects of the invention will vary if the lectin has been denatured or destabilised in any way.

The concentrations of *Robinia pseudoacacia* lectins which have been provided in this text are based on their natural properties with effectively no reduction of their activity by denaturation or destabilisation. Thus, the concentrations of lectins which are provided are not absolute but reflect the activity of the lectin. Accordingly, a composition, for example, which has a lectin whose activity has been reduced by half, but which is present in double the concentration is the same as a composition which has half the quantity of lectin but where the activity of the lectin has not been altered. Furthermore, the activity of any lectin may be increased by modification, for example during recombinant production and/or by producing truncated mutants which have increased activity. The same considerations as to the concentration verses activity of lectin also applies to lectins with increased activity. All modified *Robinia pseudoacacia* lectins or derivatives thereof are covered by the present invention, including those with increased or decreased activities, and include, for example, truncated lectin subunits and/or differently glycosylated versions with full or modified activity. Also included are protein sequences which include one or more of the critical amino acids involved in biological activity of any lectin (preferably all of those amino acids and all of the biological activity). For example, the active region(s) of RPA polypeptide b sequence are described in Nishiguchi *et al*, 1997, and include the following; (amino acid: position); aspartate: 87, phenylalanine: 130, threonine: 215, leucine: 217, serine: 218.

The *Robinia pseudoacacia* lectin may be from, or is an equivalent to a lectin from any part of the *Robinia pseudoacacia* plant, preferably bark, seed, root, root nodule, phloem or wood or a combination of two or more thereof. In accordance with the present invention, the *Robinia pseudoacacia* lectin may comprise only one polypeptide subunit The polypeptide subunit may be any of those known in the art (e.g. RPbAI (a or b), RPbAII(c), RPsAI or RPsAII, RprAI or RPrAII) or an equivalent thereto, or is a polypeptide subunit not yet identified. Preferably, the *Robinia pseudoacacia* lectin of the first aspect of the invention comprises a monomer, a dimer, a trimer or a tetramer of subunits, or a combination of two or more thereof. This enables mimicking of polypeptide groupings as they occur in nature. The monomer, dimer, timer, tetramer may be of the same, or more than one type of polymer subunit The tetramer preferably comprises four bark polypeptide a subunits, four bark polypeptide b subunits or an intermediate mixture of a and b subunits. The lectin polypeptides may comprise or correspond to one or more of the amino acid sequences as set out in Figure 1 or Figure 2, preferably at least 40, 50, 60, 70, 80, 90, 100% homologous thereto (as a lectin variant as described above).

The homotetramer, a homotrimer, a homodimer or a single subunit, is preferably purified to a level of 60% or more. In this text, the term "purified" means substantially without impurities. *Robinia pseudoacacia* lectin at these levels of purity is preferred for use in pharmaceutical compositions and use in medicine. The *Robinia pseudoacacia* lectin is preferably purified to a level of 70, 80, 90%, or above, most preferably 95%, 99% or 100%.

The RPA lectin may be in combination with a pharmaceutically acceptable excipient. Preferably the composition is a pharmaceutical composition which comprises a pharmaceutically acceptable excipient. Suitable excipients include pharmaceutical grade starch, gelatin, oil, sugar, alcohol or water (preferably sterile). The composition may be a mixed preparation for administration, or may be a combined preparation for simultaneous, separate or sequential use (or administration). In a combined preparation, the lectin and any other components may be separate and may be administered in any order. Features and preferred features of the previous aspects of the invention as described above also apply to this aspect. In particular, the composition may comprise a *Rabinia pseudoacacia* lectin in combination with a cytoprotectant as hereinbefore described.

Another aspect of the invention provides the use of *Robinia pseudoacacia* lectin in the manufacture of a medicament for the control of mucosal cell proliferation, for the reduction and/or treatment of damage caused by a cell-damaging agent, for the reduction and/or treatment of a metabolic disorder or a combination thereof. Features and preferred features of previous aspects of the invention also apply to this aspect.

The medicament can be administered to an individual in need thereof in particular where they will undergo, are undergoing or have undergone radio and/or chemotherapy and, in particular, if they are suffering from cancer.

A further aspect of the invention provides a method to produce the lectin as described above. Production of the purified lectin involves either the isolation and purification of *Robinia pseudoacacia* lectin from *Robinia pseudoacacia* (eg from a plant or tissue culture) or to the synthetic production (eg by recombinant technology). Production of a composition involves combining the lectin with any one or more ingredients for the composition. Preferred features of the previous aspects also apply to this aspect.

For each of the aspects of the invention which may be involved in medicine or involve any administration to biological tissue, the particular dosage regime will ultimately be determined by the attending physician and will take into consideration such factors as the lectin or lectins being used, animal type, age, weight, severity of symptoms and/or severity of treatment being or to be applied, method of administration, adverse reactions and/or other contraindications. Specific defined dosage ranges can be determined by standard designed clinical trials with patient progress and recovery being fully monitored. Such trials may use an escalating dose design using a low percentage of the maximum tolerated dose in animals as the starting dose in man. Preliminary guidance for dosage ranges can be taken from the results given in the experimental section of this text and by the following ranges which have been extrapolated to humans.

An appropriate upper limit appears to be a concentration of a lectin up to approximately 0.3 g per kg body weight per day. A concentration of 0.3 g per kg body weight per day may well produce an upset gastro-intestinal tract in a patient but these symptoms may be acceptable on the basis that the patient has an increase chance of survival from diseases such as cancer. An appropriate lower limit appear to be a concentration of lectin around 0.0001 mg (0.1 µg) per kg body weight per day. Preferred intermediate dosage concentrations include the presence of a lectin up to approximately 0.2 g, 0.15 g and 0.05 g per kg body weight per day, and thereafter of concentrations approximately 1 mg, 0.5 mg, 0.1 mg, 0.01 mg and 0.005 mg per kg body weight per day.

The medicaments for all aspects of the present invention can be used to provide the required concentration of lectin in one or more "intake" per day ("intake" including any form of administration and also describing a dosage unit). Some aspects of the invention may suit a single intake of a high lectin concentration, whereas others may suit a number of intakes, evenly or unevenly spaced over the same period, but with a lower lectin concentration per intake.

Use of the lectin in any medicament (including the manufacture of medicaments) according to the present invention may be in combination with a suitable pharmaceutical carrier and/or excipient. Such carriers and excipients are well known in the art (eg Handbook of Pharmaceutical Excipients (1994) 2^{nd} Edition. Eds. A. Wade/P.J. Weller. The Pharmaceutical Press, American Pharmaceutical Association). Of particular use according to the present invention are compositions and/or medicaments which are formulated in a colonic drug delivery system, a mouthwash, a cream, a paste or in a capsule.

The medicament manufactured according to any aspect of the invention is preferably administered by mouth (orally), nasally (for example via a nasal spray) or rectally (for ease of route to one or more parts of the alimentary canal) although parenteral or intravenous administration of the medicament may also be used.

The present invention will now be illustrated by the following examples.

### EXAMPLES

### Preparation of lectin from the bark of Robinia pseudoacacia

The Robinia bark lectins may be prepared essentially as described by Van Damme *et al*., (1995 a) from partially purified bark extracts on immobilised fetuin.

Bark tissue was homogenised in 20mM acetic acid containing 200 mg/l ascorbic acid (10 ml/g fresh weight) using a blender. The homogenate was squeezed through cheesecloth, adjusted to pH 5.0 (with 1M NaOH), and centrifuged for 10,000g for 10 minutes. The resulting supernatant was filtered through glass wool to remove floating particles. After adding 1.5 g/l CaCl₂, the extract was brought to pH 9.0 (with 1M NaOH) and kept overnight at 4°C. The resulting precipitate was removed by centrifugation and the cleared extract adjusted to pH 3.8 with 1m HCl. After standing overnight again at 4°C, the extract was cleared by centrifugation and adjusted to pH 7.0 with 1M NaOH and brought to 1M ammonium sulphate by adding the solid salt. The solution was then degassed and recentrifuged. The cleared supernatant was loaded onto a fetuin agarose column equilibrated with 1M ammonium sulphate (pH 7.0). After passing the extract, the column was washed with ammonium sulphate until the A₂₈₀ fell below 0.01. The lectin was. then desorbed with 20mM diaminopropane (pH 11). The desorbed lectin was adjusted to pH 7.0 with 1M HCI and bought to 1M ammonium sulphate by adding the solid salt. After centrifuging at 15.000g for 20 minutes, the supernatant was loaded on a column of phenyl-Sepharose equilibrated with 1M ammonium sulphate. Impurities were removed by washing with 1M ammonium sulphate and the lectin desorbed using a linear gradient of 1M ammonium sulphate to water. Fractions were collected and the A₂₈₀ determined to identify those containing lectin.

It is known that the bark lectin is produced *by Robinia pseudoacacia* in response to shortening day length i.e. the onset of winter. By using bark material in winter, yields of lectin in the region of 10 mg/g may be attained.

### Preparation of A₄ and B₄ RPA bark lectin isotypes

The A₄ and B₄ homotetramer isotypes may be prepared using a step gradient ion exchange chromatography method. A 100 ml Hyper-D S column (BioSepra) was equilibrated with five column volumes of 50 mM sodium acetate pH 4.75. Freeze dried RPA lectin (obtained as described in the above preparation) was dissolved in acetate buffer (50mM sodium acetate, pH 4.75) and centrifuged for 10 minutes at 20,000g. The clarified supernatant was applied to the column and the column subsequently washed with acetate buffer at a flow rate of 3 ml/min. A step gradient was constructed using 50 mM sodium acetate pH 4.75 + NaCl. The NaCl was added in increasing concentrations in 5, 7, 9, 12 and 100% step gradients. The fractions containing lectin were selected by measuring the absorbance at 280 nM. The A₄ tetramer was eluted in the 5.7% salt fraction and the B₄ tetramer in the 9-12% salt fraction.

The fractions containing pure A₄ or B₄ were confirmed using conventional PAGE and SDS-PAGE methods.

### Characterisation of lectins from the bark of Robinia pseudoacacia

The lectins from the bark of *Robinia pseudoacacia* can be characterised further using ion exchange chromatography, gel filtration and *in vitro* cell agglutination assays. Such methods are well known in the art and are described in Van Damme *et al* 1995a and b.

### Animal management

Thirty five young male Hooded Lister (Rowett Strain) rats (19 days old weighing 30-40g) were housed individually in grid bottom cages. The animals were fed a high quality semi-synthetic diet containing 10% lactalbumin protein (Table 1).

**Table 1**

| Composition of diet. | |
|---|---|
| | (g/kg diet) |
| Maize starch | 373 |
| Potato starch | 100 |
| Glucose | 150 |
| Corn oil | 150 |
| Vitamin mix | 50 |
| Mineral mix | 50 |
| Lactalbumin | 127 |
| Silicic acid | 0.4 |

For composition of vitamin and mineral mixes see Carvalho (1992).

Diet and water was freely available at all times except on day 19 when the amount of food offered was restricted to 10g/rat. At 19 days, rat weights were 100-104g.

### Administration of RPA

Animals pre-dosed with RPA were given the equivalent of 25, 50 or 100 mg/kg body weight RPA (Table 2) in 0.9% saline by gavage. The animals were pre-dosed for three consecutive days prior to the 5-FU injection.

### Administration of saline

Animals not pre-dosed with RPA were administered with 1 ml 0.9% saline by gavage.

### Administration of 5-FU

300mg of 5-fluorouracil was stirred in 14ml of distilled water. 1M NaOH was added slowly until the 5-FU had dissolved. The solution was made up to a final volume of 20 ml. The final pH of the solution was 8.3. A dose of 150 mg/kg body weight was administered to the animal by intraperitoneal injection. Immediately after the injection, the rats were offered 15g of the control diet.

The ability of orally ingested lectins from *Robinia pseudoacacia* to protect rats from a high dose of chemotherapy, and in particular its tissue protectant effect on the gut.

Five groups, each consisting of 5 rats were fed on the standard diet containing lactalbumin (Table 1). One group of rats were fed 8 g per day of standard diet for three days and then injected with 5-FU at 150 mg/kg body weight (Table 2).

**Table 1**

| Dietary protocol | |
|---|---|
| Treatment | Dietary protocol |
| 1 | LA 3d, inject 5-FU, LA 6d |
| 2 | 25 mg/kg RPA 3d, inject 5-FU, LA 6d |
| 3 | 50 mg/kg RPA 3d, inject 5-FU, LA 6d |
| 4 | 100 mg/kg RPA 3d, inject 5-FU, LA 6d |
| 5 | 100 mg/kg RPA 3d, LA 6 d |
| Key LA = lactalbumin RPA *= Robinia pseudoacacia* lectin 5-FU = 5-Fluorouracil | |

The rats were pair fed for the first three days and then fed *ab libitum* post 5-FU injection.

Three groups of animals were gavaged daily with 25, 50 or 100 mg/kg/day body weight *Robinia pseudoacacia* lectin (RPA) for three days and then injected with 150 mg/kg 5-FU on the following day. The remaining group of animals were pre-dosed with 100 mg/kg body weight RPA and then returned to lactalbumin containing diet without injection of 5-FU. After injection with 5-FU, the animals were returned to lactalbumin containing diet *ad lib*. The animals were sacrificed 7 days after the start of the experiment, the carcass dissected and the small intestine, jejunum and ileum wet weights recorded. The results are shown in Table 3.

**Table 3.**

| The effect of pre-dosing with Robinia pseudoacacia lectin for 3 days on the small intestine, jejunum and ileum wet weights (g). | | | | |
|---|---|---|---|---|
| Treatment | Dosing regime | Small intestine (g) | Jejunum (g) | Ileum (g) |
| 1 | LA + 5-EU | 5.62 | 1.0 | 1.0 |
| 2 | 25 mg/kg RPA + 5-EU | 5.41 | 1.03 | 0.98 |
| 3 | 50 mg/kg RPA + 5-FU | 5.74 | 1.12 | 1.08 |
| 4 | 100 mg/kg RPA + 5-FU | 6.33 | 1.27 | 1.12 |
| 5 | 100 mg/kg RPA | 6.99 | 1.27 | 1.17 |

The results show a dose-dependent increase in wet weight for all tissues examined with increasing dose of RPA. Thus, pre-dosing with RPA was able to protect tissues of the small intestine, jejunum and ileum from damage induced by 5-FU. Pre-dosing with 100 mg/kg RPA (treatment 4) gave the best tissue protectant effect when compared to the animals fed lactalbumin alone (treatment 1). Animals pre-dosed with 100 mg/kg RPA and injected with 5-FU (treatment 4) had similar small intestine, jejunum and ileum wet weights to animals fed 100 mg/kg RPA alone (treatment 5).

Visual inspection of the small intestine, jejunum and ileum indicated that animals treated with 5-FU alone (treatment 1) had no, or a very thin mucosal layer on the gut tissue. Animals treated with 5-FU and RPA however had healthy looking gut tissue with a normal mucosal layer.

This example demonstrates that oral administration of *Robinia pseudoacacia* lectin (RPA) before injection with 5-FU gave a significant tissue protective effect on the small intestine, jejunum and ileum.

The ability of orally ingested homotetramer A₄ and B₄ lectins from *Robinia pseudoacacia* to protect rats from a high dose of chemotherapy, and in particular its tissue protectant effect on the gut.

Five groups, each consisting of 5 rats were fed on a standard diet containing lactalbumin (Table 1) and were fed on the following dietary regime (Table 4);

**Table 4.**

| Dietary protocol | |
|---|---|
| Treatment | Dietary protocol |
| 1 | LA 7d |
| 2 | LA 3d, inject 5-FU, LA 4d |
| 3 | 200 mg/kg A₄ homotetramer 3d, inject 5-FU, LA 4d |
| 4 | 200 mg/kg B₄ homotetramer 3d, inject 5-FU, LA 4d |

One group of animals (Treatment 1) were fed standard diet throughout. The second group were gavaged daily with saline for three days and then injected with 5-FU at 150 mg/kg body weight (Treatment 2). The remaining two groups (Treatment 3 & 4) of animals were gavaged daily for three days with either A₄ or B₄ RPA homotetramer for three days and then injected with 150 mg/kg body weight 5-FU. After injection with 5-FU, Treatment groups 2-4 were returned to standard diet for four days *ad lib.* The animals were sacrificed 7 days after the start of the experiment, the carcass dissected and the small intestine, jejunum and ileum dry weights recorded. The results are shown in Table 5.

**Table 5.**

| The effect of pre-dosing with *Robinia pseudoacacia* A₄ or B₄ homotetramer lectin for 3 days on the small intestine, jejunum and ileum dry weights (mg). | | | | |
|---|---|---|---|---|
| Treatment | Dosing regime | Small Intestine (mg) | Jejunum (mg) | Ileum (mg) |
| 1 | LA | 2531 | 519 | 415 |
| 2 | LA + 5-FU | 1789 | 336 | 405 |
| 3 | 200 mg/kg A₄ | 2597 | 632 | 421 |
| 4 | 200 mg/kg B₄ | 2093 | 439 | 419 |

Dry weights normalised to 100g dry body weight.

The results show that treatment with 5-FU had a large effect on the small intestine and in particular the jejunum. Animals treated with 5-FU had a significant reduction in tissue dry weight versus the untreated control (Treatments 1 & 2). Pre-treatment with either the A₄ or B₄ homotetramer lectin however gave a significant tissue protectant effect (Treatment 3 & 4). In particular, animals treated with the A₄ homotetramer lectin had tissue dry weights essentially the same as that of the untreated animals.

Visual inspection of the small intestine, jejunum and ileum indicated that the animals treated with 5-FU alone had extensively damaged mucosal on the gut tissue. However, the animals treated with the A₄ or B₄ homotetramer lectin had essentially a normal looking mucosal layer.

This example demonstrates that both the A₄, and B₄ homotetramer *Robinia pseudoacacia* lectin were able to give significant protective effect on the gut when administered before injection with 5-FU.

### References

Banach M., Zaremba S., Sadouska M. (1983). Disturbances of liver and muscle glycogen level as well as blood glucose level in mice following administration of lectin extracted from *Robinia pseudoacacia.* Folia Biol. (Krakow) Vol 31, pp 177-186.
Bardocz, S.. Grant, G., Ewen, S.W.B., Duguid. T.J., Brown, D.S., Englyst. K. & Pusztai, A. (1995). Reversible effect of Phytohaemagglutinin on the growth and metabolism of the rat gastrointestinal tract. Gut, 37, 353-360.
Carvalho, A.F.F.U. de, (1992). Dietary kidney bean lectins affect insulin levels. change gene expression and modulate metabolism. PhD thesis: University of Aberdeen.
Denekamp, J. (1996). The broad spectrum of preclinical radiobiology: British contributions. International Journal of Radiation Oncology, Biology & Physics, 36. 497-509.
Duverger, E. and Delmotte, F. M. Purification of lectins from *Robinia pseudoacacia* L. root-tips. Plant Science 123.9-18, (1997).
Erkisi, M., Erkurt, E., Ozbarlas. S., Burgut, R., Doran. F. & Seyrek, E. (1996). The use of recombinant human granulocyte colony-stimulating factor in combination with single or fractionated doses of isofamide and doxorubicin in patients with soft tissue sarcoma. Journal of chemotherapy, 8,224-228.
Fleischmann, G. and Rudiger, H. (1986). Isolation, resolution and partial characterisation of two *Robinia pseudoacacia* seed lectins. Biol. Chem. Hoppe-Seyler, 367, 27-32.
Gietl, C. and Ziegler H., Biochem, Physiol. Planzen 175, 58-66 (1080)
Lehninger, AL., Nelson, D.L. & Cox, M.C. (1997). Principles of biochemistry. Worth Publishers, New York.
Nishiguchi, M., Yoshida, K., Sumizono, T. & Tazaki, T. (1997). Studies by site directed mutagenesis of the carbohydrate binding properties of a bark lectin from Robinia pseudoacacia. FEBS Letters, 403, 294-298.
Paulsen, F., Hoffmann, W., Kortmann, R.D., Porschen, R. & Bamberg, M. (1996). Akute gastrointestinal Nebenwirkungen in der Radio-onkologie - Was ist gesichert in der Therapie?. Strahlenther. Onkol. 172. 53-56 (Nr 2).
Podolsky, D.K. (1993). Regulation of intestinal epithelial proliferation: a few answers, many questions.
Pusztai, A. (1991). Plant Lectins. Cambridge: Cambridge University Press.
Pusztai, A. (1993) Dietary lectins are metabolic signals for the gut and modulate immune and hormone functions. Eur J of Clin Nut, 47, 691-699.
Pusztai, A., Ewan, S.W.B., Grant G., Peumans, W.J., Van Damme, E.J.M., Coates, M.E. and Bardocz, S. (1995). Lectins and also bacteria modify the glycosylation of gut receptors in the rat. Glycoconjugate Journal, 12, 22-35.
Raedler, A., Boehle A., Otto, U. & Raedler, E. (1982). Differences of glycoconjugate exposed on hypemephroma and normal kidney cells. Journal of Urology, 128, 1109-1113.
Sabeur, G., Wantyghem, J & Schuller, E. (1986). Stimulation of 2',3'-cyclic nucleotide 3'-phosphodiesterase in human lymphocytes by Robinia pseudoacacia lectin. Biochemie, 68, 581-585.
Sharif, A., Brochier, J. & Bourrillon, R. (1977). Specific activation of Human T lyphocytes by Robinia pseudoacacia seeds lectin. Cellular Immunology, 31, 302-310.
Sharif, A., Lethibichthuy, J., Brochier, J., Goussalt, Y. & Bourrillon, R. (1978). Activation of human B lymphocytes induced by Robinia pseudoacacia lectin in the presence of T cells. Immunology, 35, 643-649.
Steel, G.G. (1996). From targets to genes: a brief history of radiosensitivity. Phys Med. Biol., 41, 205-222.
Van Damme, E.J.M., Barre, A., Smeets, K., Torrekens, S., Van Leuven, F., Rouge, P. & Peumans, W.J. (1995a). The bark of *Robinia pseudoacacia* contains a complex mixture of lectins. Plant Physiol., 107, 833-843.
Van Damme, E.J.M., Barre. A., Rouge, P., Van Leuven, F., and Peumaas, W. (1995b). The seed lectins of black locust (*Robinia pseudoacacia*) are encoded by two genes which differ from the bark lectin genes. Plant Molecular Biology, 29, 1197-1210.
Van Halteren, H.K., Gortzak, E., Taal, B.G., Helmerhorst, Th, J, M., Aleman, B.M.P., Hart, A.A.M. & Zoetmulder, F.A.N. (1993). Surgical intervention for complications caused by late radiation damage of the small bowel: a retrospective analysis. European Journal of Surgical Oncology, 19, 336-341.
Wantyghem, J., Goulut, C., Frenoy, J.P., Turpin. E. and Goussault, Y. (1986). Purification and characterisation of *Robinia pseudoacacia* seed lectins. A re-investigation. Biochem J., 237, 483-489.
Yeoh, E., Horowitz, M., Russo, A., Muecke, T., Ahmad, A. & Chatterton, B. (1993). International Journal of Radiation Oncology, Biology & Physics, 26, 229-237.
Yoshida, K., Baba, K., Yamamoto, N. & Tazaki, K (1994). Cloning of a lectin cDNA and seasonal changes in levels of the lectin and its mRNA in the inner bark of Robinia pseudoacacia. Plant Mol. Biol. (1994), 25, 845-853.

## Claims

1. Use of *Robinia pseudoacacia* lectin in the manufacture of a medicament for the control of mucosal cell proliferation, for the reduction and/or treatment of damage caused by a cell-damaging agent, or a combination thereof.

2. Use of *Robinia pseudoacacia* lectin as claimed in claim 1, wherein the medicament is administered to a patient undergoing, having undergone, or will undergo radio and/or chemotherapy, in particular wherein the patient is suffering from cancer.

3. Use of *Robinia pseudoacacia* lectin, as claimed in claims 1 or 2, wherein the use is for the reduction and/or treatment of mucositis or gut lesions.

4. Use of *Robinia pseudoacacia* lectin, as claimed in claim 1, wherein the use is for the reduction and/or treatment of a bowel disorder.

5. Use as claimed in claim 4, wherein the bowel disorder is inflammatory bowel disease or irritable bowel syndrome.

6. Use, as claimed in any one of claims 1 to 5, wherein the *Robinia pseudoacacia* lectin comprises bark, seed, root, root nodule, phloem, wood or leaf lectin or a combination of two or more thereof.

7. Use, as claimed in any one of claims 1 to 6, wherein the lectin comprises a monomer, dimer, trimer or tetramer of subunit(s) or a combination of two or more thereof.

8. Use, as claimed in claim 7, wherein the lectin comprises a homopolypeptide.

9. Use, as claimed in any one of claims 1 to 8, wherein the lectin is a homotetramer of bark subunits a or b.

10. Use, as claimed in any one of claims 1 to 9, wherein the lectin is a homotetramer, a homotrimer, a homodimer, a single subunit or a combination thereof purified to a level of 60% or more, optionally bark subunits a or b or in a composition as claimed in claim 21 or 22.

11. Use as claimed in any one of claims 1 to 10, for mammalian cells and/or tissues, in particular human cells and/or tissues.

12. Use as claimed in claim 11, wherein the cell and/or tissue is a mucous cell, including a mucous membrane.

13. *Robinia pseudoacacia* lectin consisting of a homotetramer, homotrimer, homodimer, single subunit or a combination of two or more thereof, of bark a subunit for use in medicine.

14. *Robinia pseudoacacia* lectin consisting of a homotetramer, homotrimer, homodimer, single subunit, or a combination of two or more thereof, of bark b subunit for use in medicine.

15. *Robinia pseudoacacia* lectin as claimed in any one of claim 13 or claim 14, for use in the treatment of a patient undergoing, having undergone, or will undergo a radio and/or chemotherapy, in particular wherein the patient is suffering from cancer.

16. *Robinia pseudoacacia* lectin as claimed in any one of claims 13 to 15, wherein the use is for the control of mucosal cell proliferation, for the reduction and/or treatment of damage caused by a cell-damaging agent or a combination thereof.

17. *Robinia pseudoacacia* lectin as claimed in claim 16, wherein the use is for the control or reduction and/or treatment of mucositis or gut lesions.

18. *Robinia pseudoacacia* lectin, as claimed in claim 16, wherein the use is for the reduction and/or treatment of a bowel disorder.

19. *Robinia pseudoacacia*, as claimed in claim 18, wherein the bowel disorder is inflammatory bowel disease or irritable bowel syndrome.

20. *Robinia pseudoacacia* lectin as claimed in any one of claims 13 to 19, for mammalian cells and/or tissues, in particular human cells and/or tissues.

21. *Robinia pseudoacacia* lectin as claimed in claim 20, wherein the cell and/or tissue is a mucous cell, including a mucous membrane.

22. *Robinia pseudoacacia* lectin in combination with a cytoprotectant for use in medicine.

## Patentansprüche

1. Verwendung von *Robinia pseudoacacia* Lectin in der Herstellung eines Medikaments zur Kontrolle der Proliferation von Schleimhautzellen, zur Reduktion und/oder Behandlung von Schädigungen, die durch eine zellschädigende Substanz hervorgerufen wurden, oder eine Kombination davon.

2. Verwendung von *Robinia pseudoacacia* Lectin nach Anspruch 1, wobei das Medikament einem Patienten verabreicht wird, der sich gerade einer Radio- und/ oder Chemotherapie unterzieht, sich einer unterzogen hat, oder sich einer unterziehen wird, insbesondere wenn der Patient an Krebs leidet.

3. Verwendung von *Robinia pseudoacacia* Lectin nach Anspruch 1 oder 2, wobei die Verwendung zur Reduktion und/oder Behandlung von Mucositis oder Darmläsionen besteht.

4. Verwendung von *Robinia pseudoacacia* Lectin nach Anspruch 1, wobei die Verwendung in der Reduktion und/oder Behandlung von einer Funktionsstörung der Eingeweide bestimmt ist.

5. Verwendung nach Anspruch 4, wobei die Funktionsstörung der Eingeweide eine entzündliche Erkrankung der Eingeweide oder ein reizbares Eingeweide-Syndrom ist.

6. Verwendung nach einem der Ansprüche 1 bis 5, wobei das *Robinia pseudoacacia* Lectin Rinde, Samen, Wurzel, Wurzel-Knötchen, Phloem, Holz oder Blatt-Lectin oder eine Kombination zweier oder mehrer davon enthält.

7. Verwendung nach einem der Ansprüche 1 bis 6, wobei das Lectin ein Monomer, Dimer, Trimer oder Tetramer von (einer) Untereinheit(en) oder eine Kombination zweier oder mehrerer davon enthält.

8. Verwendung nach Anspruch 7, wobei das Lectin ein Homopolypeptid enthält.

9. Verwendung nach einem der Ansprüche 1 bis 8, wobei das Lectin ein Homotetramer der Rinde-Untereinheiten a oder b ist.

10. Verwendung nach einem der Ansprüche 1 bis 9, wobei das Lectin ein Homotetramer, ein Homotrimer, ein Homodimer, eine einzelne Untereinheit oder eine Kombination davon, gereinigt bis zu einem Grad von 60% oder mehr, gegebenenfalls Rinden-Untereinheiten a oder b oder in einer Zusammensetzung nach Anspruch 21 oder 22 ist.

11. Verwendung nach einem der Ansprüche 1 bis 10, für Säugetierzellen und/oder Gewebe, insbesondere menschliche Zellen und/oder Gewebe.

12. Verwendung nach Anspruch 11, wobei die Zelle und/oder das Gewebe eine Schleim-Zelle, einschließlich einer Schleimhaut ist.

13. *Robinia pseudoacacia* Lectin, welches aus einem Homotetramer, Homotrimer, Homodimer, einer einzelnen Untereinheit oder einer Kombination zweier oder mehrerer davon und aus der Rinden Untereinheit a besteht, zur Verwendung in der Medizin.

14. *Robinia pseudoacacia* Lectin, welches aus einem Homotetramer, Homotrimer, Homodimer, einer einzelnen Untereinheit oder einer Kombination zweier oder mehrerer davon und aus der Rinden Untereinheit b besteht, zur Verwendung in der Medizin.

15. *Robinia pseudoacacia* Lectin nach einem der Ansprüche 13 oder 14, zur Verwendung in der Behandlung eines Patienten, der sich gerade einer Radio- und/oder Chemotherapie unterzieht, sich einer unterzogen hat, oder sich einer unterziehen wird, insbesondere wenn der Patient an Krebs leidet.

16. *Robinia pseudoacacia* Lectin nach einem der Ansprüche 13 bis 15, wobei die Verwendung in der Kontrolle der Proliferation von Schleimhautzellen, in der Reduktion und/ oder Behandlung von Schädigungen, die durch eine zellschädigende Substanz hervorgerufen wurden, oder einer Kombination davon besteht.

17. *Robinia pseudoacacia* Lectin nach Anspruch 16, wobei die Verwendung in der Kontrolle oder Reduktion und/oder Behandlung von Mucositis oder Darmläsionen besteht.

18. *Robinia pseudoacacia* Lectin nach Anspruch 16, wobei die Verwendung in der Reduktion und/oder Behandlung von einer Funktionsstörung der Eingeweide besteht.

19. *Robinia pseudoacacia* Lectin nach Anspruch 18, wobei die Funktionsstörung der Eingeweide eine entzündliche Erkrankung der Eingeweide oder ein reizbares Eingeweide-Syndrom ist.

20. *Robinia pseudoacacia* Lectin nach einem der Ansprüche 13 bis 19, für Säugetierzellen und/oder Gewebe, insbesondere menschliche Zellen und/oder Gewebe.

21. *Robinia pseudoacacia* Lectin nach Anspruch 20, wobei die Zelle und/oder das Gewebe eine schleimartige Zelle, einschließlich einer Schleimhaut ist.

22. *Robinia pseudoacacia* Lectin in Kombination mit einem Zellschutz (Cyto-Protector) zur Verwendung in der Medizin.

## Revendications

1. Utilisation de lectine de *Robinia pseudoacacia* dans la fabrication d'un médicament pour le contrôle de la prolifération de cellules muqueuses, pour la réduction et/ou le traitement de dommages causés par un agent endommageant des cellules, ou pour une combinaison de ceux-ci.

2. Utilisation de lectine de *Robinia pseudoacacia* selon la revendication 1, dans laquelle le médicament est administré à un patient subissant, ayant subi ou qui va subir une radio- et/ou une chimiothérapie, en particulier à un patient qui souffre d'un cancer.

3. Utilisation de lectine de *Robinia pseudoacacia* selon les revendications 1 ou 2, l'utilisation servant à la réduction et/ou au traitement d'une inflammation d'une muqueuse ou de lésions intestinales.

4. Utilisation de lectine de *Robinia pseudoacacia* selon la revendication 1, l'utilisation servant à la réduction et/ou au traitement d'une affection intestinale.

5. Utilisation selon la revendication 4, dans laquelle l'affection intestinale est une maladie inflammatoire de l'intestin ou un syndrome d'intestin irritable.

6. Utilisation selon l'une quelconque des revendications 1 à 5, dans laquelle la lectine de *Robinia pseudoacacia* comprend la lectine d'écorce, de graine, de racine, de nodule de racine, de phloème, de bois ou de feuille, ou une combinaison de deux ou plusieurs de celles-ci.

7. Utilisation selon l'une quelconque des revendications 1 à 6, dans laquelle la lectine comprend un monomère, un dimère, un trimère ou un tétramère de sous-unité(s) ou une combinaison de deux ou plusieurs de ceux-ci.

8. Utilisation selon la revendication 7, dans laquelle la lectine comprend un homopolypeptide.

9. Utilisation selon l'une quelconque des revendications 1 à 8, dans laquelle la lectine est un homotétramère de sous-unités d'écorce a ou b.

10. Utilisation selon l'une quelconque des revendications 1 à 9, dans laquelle la lectine est un homotétramère, un homotrimère, un homodimère, une seule sous-unité ou une combinaison de ceux-ci purifiés à un niveau de 60% ou plus, facultativement des sous-unités d'écorce a ou b, ou dans une composition selon la revendication 21 ou 22.

11. Utilisation selon l'une quelconque des revendications 1 à 10, pour des cellules et/ou des tissus de mammifères, en particulier des cellules et/ou des tissus humains.

12. Utilisation selon la revendication 11, dans laquelle la cellule et/ou le tissu est une cellule muqueuse, y compris une membrane muqueuse.

13. Lectine de *Robinia pseudoacacia* constituée d'un homotétramère, d'un homotrimère, d'un homodimère, d'une seule sous-unité, ou d'une combinaison de deux ou plusieurs de ceux-ci, d'une sous-unité d'écorce a pour l'utilisation en médecine.

14. Lectine de *Robinia pseudoacacia* constituée d'un homotétramère, d'un homotrimère, d'un homodimère, d'une seule sous-unité, ou d'une combinaison de deux ou plusieurs de ceux-ci, d'une sous-unité d'écorce b pour l'utilisation en médecine.

15. Lectine de *Robinia pseudoacacia* selon l'une quelconque des revendications 13 ou 14, pour une utilisation dans le traitement d'un patient subissant, ayant subi ou qui va subir une radio- et/ou une chimiothérapie, en particulier à un patient qui souffre d'un cancer.

16. Lectine de *Robinia pseudoacacia* selon l'une quelconque des revendications 13 à 15, dans laquelle l'utilisation sert au contrôle de la prolifération de cellules muqueuses, à la réduction et/ou au traitement de dommages causés par un agent endommageant des cellules, ou à une combinaison de ceux-ci.

17. Lectine de *Robinia pseudoacacia* selon la revendication 16, dans laquelle l'utilisation sert au contrôle ou à la réduction et/ou au traitement d'une inflammation d'une muqueuse ou de lésions intestinales.

18. Lectine de *Robinia pseudoacacia* selon la revendication 16, dans laquelle l'utilisation sert à la réduction et/ou au traitement d'une affection intestinale.

19. Lectine de *Robinia pseudoacacia* selon la revendication 18, dans laquelle l'affection intestinale est une maladie inflammatoire de l'intestin ou un syndrôme d'intestin irritable.

20. Lectine de *Robinia pseudoacacia* selon l'une quelconque des revendications 13 à 19, pour des cellules et/ou des tissus de mammifères, en particulier des cellules et/ou des tissus humains.

21. Lectine de *Robinia pseudoacacia* selon la revendication 20, dans laquelle la cellule et/ou le tissu est une cellule muqueuse, y compris une membrane muqueuse.

22. Lectine de *Robinia pseudoacacia* en combinaison avec un cytoprotecteur pour une utilisation en médecine.
